# EUROPEAN PATENT APPLICATION

(11) **EP 4 563 156 A1**
(43) Date of publication of application: **04.06.2025**
(21) Application number: 23846662.7
(22) Date of filing: 28.07.2023
(51) Int. Cl.: A61K 35/13, A61K 9/00, A61K 39/00, A61P 35/00, C12N 5/09

(54) **COMPOSITION THAT INCLUDES CANCER CELLS AND THAT IS ADMINISTERED BY A SYRINGE**

(30) Priority: 28.07.2022 JP 2022120918
(71) Applicant: Daicel Corporation, Osaka-shi, Osaka 530-0011 (JP); OSAKA UNIVERSITY, Suita-shi Osaka 565-0871 (JP)
(72) Inventor: CHANG, Chin-Yang, Suita-shi, Osaka 565-0871 (JP); TAI, Jiayu, Suita-shi, Osaka 565-0871 (JP); YAMASHITA, Kunihiko, Suita-shi, Osaka 565-0871 (JP)
(74) Representative: Grünecker Patent- und Rechtsanwälte PartG mbB
(86) International application number: PCT/JP2023/027706
(87) International publication number: WO 2024/024932

(57) **Abstract**

It was found that administering a composition containing cancer cells that express damage-associated molecular patterns (DAMPs) to a subject with a needleless injector causes the cancer cells in the composition to be damaged by shear force at the same time as the administration, and causes the DAMPs to be released from the cancer cells to the outside of the cells, whereby cellular immunity is efficiently activated.

## Description

### Technical Field

The present disclosure relates to a composition that contains cancer cells and is administered with an injector.

### Background Art

A technique to be used in cancer treatment has been developed, in which cancer cells are inactivated and the inactivated cancer cells are administered as an antigen to a subject to activate cellular immunity to cancer in the subject (Non-Patent Literature 1). Examples of the treatment using this technique include OncoVax (registered trademark) of Vaccinogen Inc., which is undergoing clinical trials as a cancer vaccine. OncoVax (registered trademark) is prepared by inactivating, through radiation treatment, cancer cells in a cancer tissue removed from a patient, and formulating the inactivated cancer cells together with a BCG adjuvant for administration. Administering this formulation to a cancer patient with a needle-equipped injector is expected for the patient to acquire immunity to the original cancer of the patient.

However, using such a technique poses problems as exemplified below.
(1) Only administration of radiation-treated cancer cells using a needle-equipped injector does not effectively release the cellular contents of the cancer cells to the outside of the cells. Thus, immune cells are less likely to be stimulated, and cellular immunity is less likely to be activated. Therefore, to facilitate activation of cellular immunity, an adjuvant intended to activate cellular immunity, such as BCG, is required to be added to the inactivated cancer cells.
(2) To completely inactivate cancer cells by radiation treatment, high-dose radiation treatment is required depending on the type of cancer cells.

### Citation List

### Patent Document

Patent Document 1: JP 2012-061269 A

### Non-Patent Literature

Non-Patent Literature 1: Cancer research 1979; 39: 1365-1360

### Summary of Invention

### Technical Problem

An object of the present disclosure is to provide a composition that contains at least cancer cells and is administered with a needleless injector.

### Solution to Problem

The present inventors have found that administering a composition containing cancer cells that express damage-associated molecular patterns (DAMPs) to a subject with a needleless injector causes the cancer cells in the composition to be damaged by shear force at the same time as the administration, and causes the DAMPs to be released from the cancer cells to the outside of the cells, whereby cellular immunity is efficiently activated. The present inventors have also found that combination with efficient delivery of a substance (antigen) into the cells by the administration with a needleless injector enables more efficient activation of cellular immunity.

Accordingly, the present disclosure is as follows.
[1] A composition containing cancer cells that express damage-associated molecular patterns (DAMPs), the composition being administered to a subject with a needleless injector.
[2] The composition according to [1], wherein the DAMPs contain at least HMGB1.
[3] A composition containing inactivated cancer cells, the composition being administered to a subject with a needleless injector.
[4] The composition according to [3], wherein the cancer cells are inactivated by radiation exposure, drug administration, or heat treatment.
[5] The composition according to [4], wherein the cancer cells are inactivated by radiation exposure and heat treatment.
[6] The composition according to any of [3] to [5], wherein the cancer cells are cultured for a certain period after the inactivation.
[7] The composition according to any of [1] to [6], wherein the cancer cells contained in the composition are cancer cells derived from a solid cancer.
[8] The composition according to [7], wherein the solid cancer is pancreatic cancer, brain tumor, pharyngeal cancer, colorectal cancer, cervical cancer, prostate cancer, breast cancer, ovarian cancer, malignant melanoma, lung adenocarcinoma, liver cancer, or renal cancer.
[9] The composition according to any of [1] to [8], wherein the cancer cells are cancer cells collected from the subject.
[10] The composition according to any of [1] to [9], wherein the composition is for treating or preventing a cancer.
[11] The composition according to [10], wherein the cancer to be treated or prevented is a cancer from which the cancer cells contained in the composition are derived.
[12] The composition according to any of [1] to [9], wherein the composition is for activating cellular immunity to cancer.
[13] A kit for treating or preventing a cancer, the kit including a needleless injector and the composition described in any of [1] to [12].
[14] A composition containing cancer cells inactivated by radiation exposure and heat treatment, the composition being administered to a subject with a needle-equipped injector.
[15] A kit for treating or preventing a cancer, the kit including a needle-equipped injector and the composition described in [14].
[16] A method for treating or preventing a cancer, the method including administering a composition containing cancer cells that express damage-associated molecular patterns (DAMPs) to a subject with a needleless injector.
[17] A method for treating or preventing a cancer, the method including administering a composition containing inactivated cancer cells to a subject with a needleless injector.
[18] A method for activating cellular immunity to cancer, the method including administering a composition containing cancer cells that express damage-associated molecular patterns (DAMPs) to a subject with a needleless injector.
[19] A method for activating cellular immunity to cancer, the method including administering a composition containing inactivated cancer cells to a subject with a needleless injector.
[20] A composition for use in treating or preventing a cancer, the composition containing cancer cells that express damage-associated molecular patterns (DAMPs), and being administered to a subject with a needleless injector.
[21] A composition for use in treating or preventing a cancer, the composition containing inactivated cancer cells, and being administered to a subject with a needleless injector.
[22] A method for treating or preventing a cancer, the method including administering a composition containing cancer cells inactivated by radiation exposure and heat treatment to a subject with a needle-equipped injector.
[23] A composition for use in treating or preventing a cancer, the composition containing cancer cells inactivated by radiation exposure and heat treatment, and being administered to a subject with a needle-equipped injector.
[24] Use of a composition containing cancer cells inactivated by radiation exposure and heat treatment in manufacturing a medicine for treating or preventing a cancer, the medicine being administered to a subject with a needle-equipped injector.
[25] A composition for use in activating cellular immunity to cancer, the composition containing cancer cells that express damage-associated molecular patterns (DAMPs), and being administered to a subject with a needleless injector.
[26] A composition for use in activating cellular immunity to cancer, the composition containing inactivated cancer cells, and being administered to a subject with a needleless injector.
[27] Use of a composition containing cancer cells that express damage-associated molecular patterns (DAMPs) in manufacturing a medicine for treating or preventing a cancer, the medicine being administered to a subject with a needleless injector.
[28] Use of a composition containing inactivated cancer cells in manufacturing a medicine for treating or preventing a cancer, the medicine being administered to a subject with a needleless injector.
[29] Use of a composition containing cancer cells that express damage-associated molecular patterns (DAMPs) in manufacturing a medicine for activating cellular immunity to cancer, the medicine being administered to a subject with a needleless injector.
[30] Use of a composition containing inactivated cancer cells in manufacturing a medicine for activating cellular immunity to cancer, the medicine being administered to a subject with a needleless injector.
[31] A method for activating cellular immunity to cancer, the method including administering a composition containing cancer cells inactivated by radiation exposure and heat treatment to a subject with a needle-equipped injector.
[32] A composition for use in activating cellular immunity to cancer, the composition containing cancer cells inactivated by radiation exposure and heat treatment, and being administered to a subject with a needle-equipped injector.
[33] Use of a composition containing cancer cells inactivated by radiation exposure and heat treatment in manufacturing a medicine for activating cellular immunity to cancer, the medicine being administered to a subject with a needle-equipped injector.

### Advantageous Effects of Invention

The present disclosure can provide a composition that contains at least cancer cells and is administered with a needleless injector.

The use of the composition according to the present disclosure provides effects as exemplified below.

First, administration of a cancer cell-containing composition to a subject with a needleless injector can efficiently activate cellular immunity, as compared with the case of administration of the same cancer-cell containing composition to the subject with a needle-equipped injector.

Furthermore, administration of the composition according to the present disclosure with a needleless injector can activate cellular immunity to cancer without using an adjuvant (e.g., BCG) intended to activate cellular immunity. That is, the composition can eliminate the use of an adjuvant commonly used in the development process of a vaccine formulation, or can reduce the amount of the adjuvant to be used. Moreover, as compared with the case of administration of a cancer cell-containing composition with a needle-equipped injector, administration of the cancer cell-containing composition with a needleless injector can predominantly activate cellular immunity and thus makes it possible to use an adjuvant other than the adjuvant intended to activate cellular immunity, such as BCG.

In the related art, in administration of cancer cells to a subject, the cancer cells are required to be completely inactivated for preventing onset of cancer. Meanwhile, in the case of administration of a composition containing cancer cells to a subject using a needleless injector, the cancer cells are damaged and killed when the cancer cells in the composition are administered into a living body. Thus, the cancer cells are not required to be completely inactivated. This led to an increase in the number of methods and conditions for inactivating cancer cells before administration of a composition containing cancer cells to a subject. Depending on the method and condition for inactivating cancer cells, cellular immunity can be efficiently activated even in the case of using a needle-equipped injector.

Furthermore, in the related art, to induce cell death of immunogenic cells, cancer cells have been exposed to an appropriate type of radiation in a living body at an intensity that induces cell death of the cancer cells. Meanwhile, the composition according to the present disclosure is, for example, a composition containing cancer cells that express DAMPs, the composition being administered to a subject with a needleless injector. That is, since the cancer cells are subjected to an optimal ex vivo inactivation treatment such as radiation exposure, drug administration, or heat treatment, cellular immunity can be efficiently activated without radiation exposure in vivo.

### Brief Description of Drawings

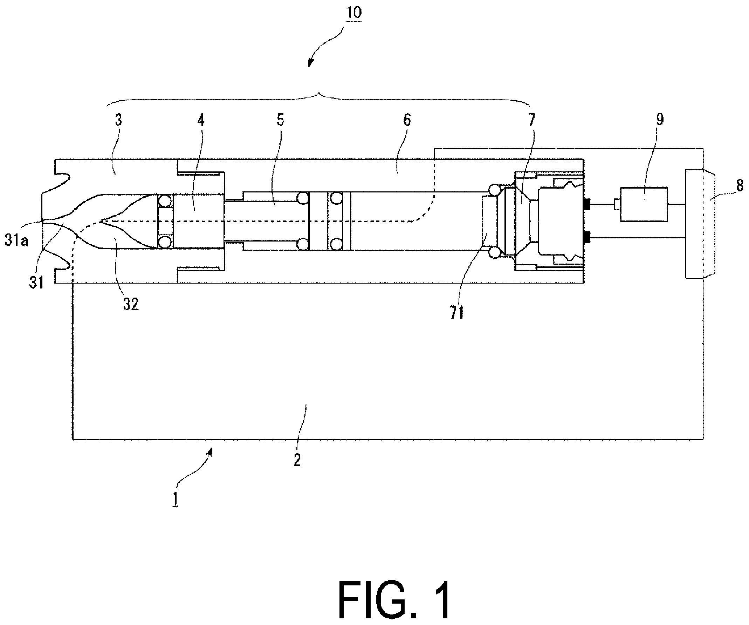

FIG. 1 is a diagram illustrating a schematic configuration of a needleless injector according to an embodiment of the present disclosure.

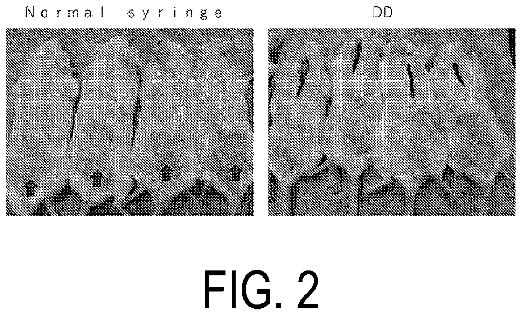

FIG. 2 shows photographs illustrating tumor development results when radiation-treated breast cancer cells were administered to mice with a needleless injector or a needle-equipped injector, according to an embodiment of the present disclosure.

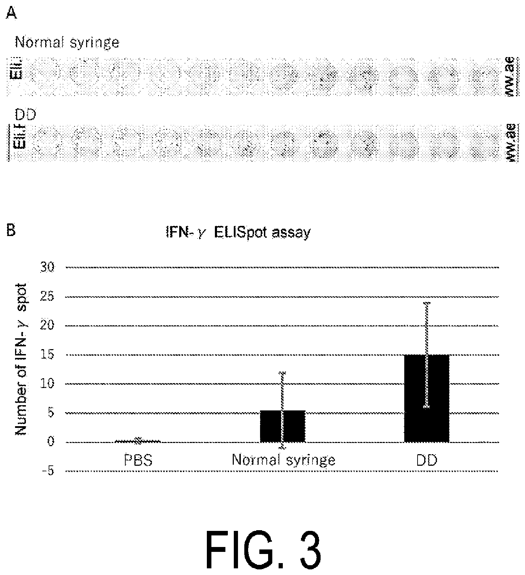

FIG. 3 shows photographs (A) and a graph (B) illustrating the results of an interferon γ ELISpot assay performed on splenocytes removed from mice administered with radiation-treated breast cancer cells with a needleless injector or a needle-equipped injector, according to an embodiment of the present disclosure.

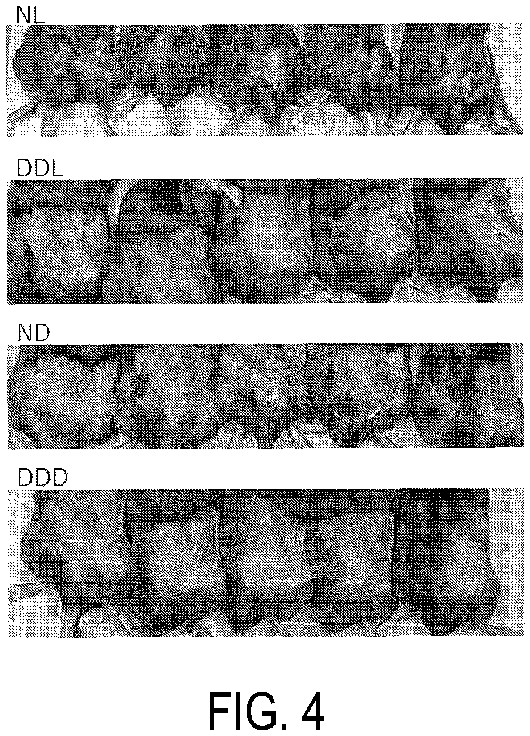

FIG. 4 shows photographs illustrating tumor development results when radiation-treated colorectal cancer cells or non-radiation-treated colorectal cancer cells were administered to mice with a needleless injector or a needle-equipped injector, according to an embodiment of the present disclosure.

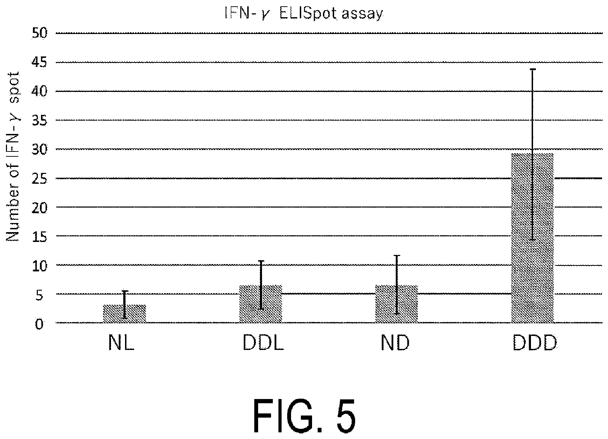

FIG. 5 is a graph illustrating the results of an interferon γ ELISpot assay performed on splenocytes removed from mice administered with radiation-treated colorectal cancer cells or non-radiation-treated colorectal cancer cells by using a needleless injector or a needle-equipped injector, according to an embodiment of the present disclosure.

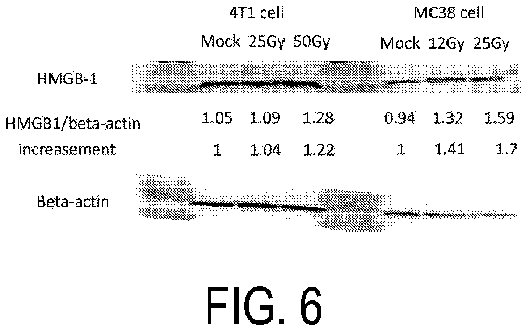

FIG. 6 shows photographs illustrating HMGB 1 expression levels after radiation exposure in various cancer cells, according to an embodiment of the present disclosure.

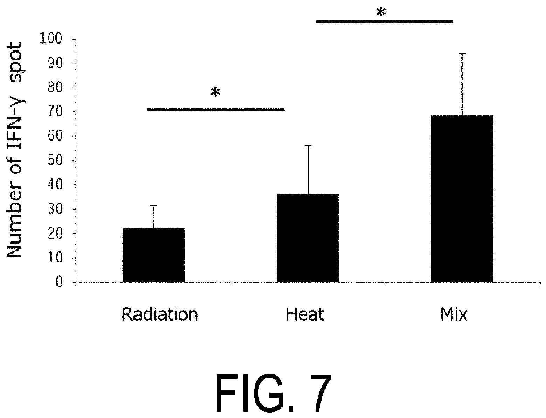

FIG. 7 is a graph illustrating the results of an interferon γ ELISpot assay performed on splenocytes removed from mice administered with colorectal cancer cells after radiation treatment, heat treatment, or combined treatment with radiation and heat by using a needle-equipped injector, according to an embodiment of the present disclosure. In the graph, * indicates p < 0.05.

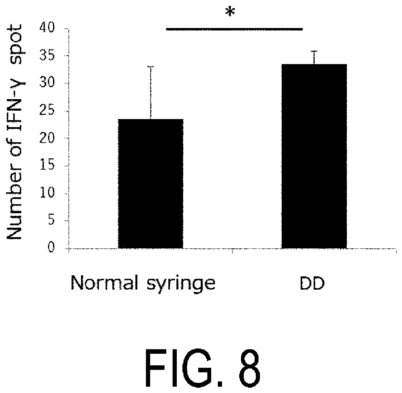

FIG. 8 is a graph illustrating the results of an interferon γ ELISpot assay performed on splenocytes removed from mice administered with radiation-treated colorectal cancer cells by using a needleless injector or a needle-equipped injector, according to an embodiment of the present disclosure. In the graph, * indicates p < 0.05.

### Description of Embodiments

Each of the configurations, a combination of the configurations, and the like in each embodiment are examples, and addition, omission, substitution, and an additional change of the configurations can be appropriately made without departing from the spirit of the present invention. The present disclosure is not limited by the embodiments and is limited only by the claims.

Each embodiment disclosed in the present specification can be combined with any other feature disclosed in the present specification.

### <Composition Containing Cancer Cells That Express Damage-Associated Molecular Patterns (DAMPs)>

An embodiment of the present disclosure is a composition containing cancer cells that express damage-associated molecular patterns (DAMPs), the composition being administered to a subject with a needleless injector.

Damage-associated molecular patterns (DAMPs) are molecules expressed by damaged cells and rarely expressed by non-damaged cells.

The cancer cells contained in the composition of the present embodiment are damaged cells, for example, cells damaged by inactivation treatment such as radiation exposure treatment. Meanwhile, non-damaged cells are typically normal cells not damaged by the inactivation treatment, but may be cancer cells not damaged by the treatment.

The cancer cells contained in the composition of the present embodiment are cells that express at least DAMPs at a higher level than non-damaged cells.

DAMPs expressed in the cancer cells contained in the composition of the present embodiment are not particularly limited as long as they do not interfere with the effects of the composition of the present embodiment. Examples of DAMPs include high-mobility group box 1 (HMGB1), heat shock proteins, S100A8, S100A9, cyclophilin A, cytochrome c, enolase-1, uric acid, matrilin-2, histone, fibrinogen, IL-33, hyaluronan, ferritin, profillin-1, fibronectin, ATP, LL-37, mtDNA, and calreticulin.

DAMPs expressed in the cancer cells contained in the composition of the present embodiment preferably contain at least HMGB1, and may contain one type or a plurality of types of DAMPs other than HMGB1. As used herein, the term "plurality" is not particularly limited as long as the effects of the composition of the present embodiment are not impaired, and the number of DAMPs other than HMGB1 may be, for example, 2 or more, 3 or more, 5 or more, or 10 or more, and may be 100 or less, 50 or less, 30 or less, or 15 or less. These lower and upper limits may be freely combined, and the number of DAMPs other than HMGB1 may be, for example, 2 or more and 100 or less, 3 or more and 50 or less, 5 or more and 30 or less, or 10 or more and 15 or less.

The level of expression of DAMPs in the cancer cells contained in the composition of the present embodiment is not particularly limited as long as the effects of the composition of the present embodiment are not impaired. The expression level of DAMPs is higher than that in control cells, and, for example, may be greater than 1.00 times, equal to or greater than 1.04 times, equal to or greater than 1.10 times, equal to or greater than 1.20 times, equal to or greater than 1.22 times, equal to or greater than 1.30 times, equal to or greater than 1.40 times, equal to or greater than 1.41 times, equal to or greater than 1.50 times, equal to or greater than 1.70 times, equal to or greater than 1.71 times, or equal to or greater than 2.00 times that in the control cells.

The needleless injector of the present embodiment is an embodiment of an injector capable of administering the composition to a subject without using an injection needle. That is, in the embodiment, the composition can be administered to a subject with an injector configured to administer a composition to a subject without using an injection needle.

Administering the composition of the present embodiment to a subject with a needleless injector achieves an effect that cellular immunity can be efficiently activated as compared with the case of using a needle-equipped injector.

That is, unlike the case of using a needle-equipped injector, the use of a needleless injector causes the cancer cells contained in the composition to be damaged by shear force at the same time as the administration, and thus DAMPs in the cancer cells are efficiently released to the outside of the cancer cells. Then, DAMPs released from the cancer cells cause local inflammation, leading to an immune response. Presumably, this results in enhanced phagocytosis of dendritic cells and efficient activation of cellular immunity by the administered composition. Thereafter, the composition provides not only a local effect at the administration site of the composition, but also activation of cellular immunity to cancer cells present at a distance from the administration site.

That is, administering the composition containing cancer cells that express DAMPs to a subject with a needleless injector can provide an effect similar to the abscopal effect with good reproducibility and can enhance the effect similar to the abscopal effect.

Thus, administering the composition of the present embodiment to a subject with a needleless injector is useful in cancer treatment, cancer prevention, and the like. An effect similar to the abscopal effect can be expected even in inoperable cases or colorectal cancer cases for which radiotherapy is impossible, and thus administering the composition of the present embodiment to a subject with a needleless injector is useful.

The cancer cells contained in the composition of the present embodiment may be derived from a solid cancer. Examples of the solid cancer include pancreatic cancer, brain tumor, pharyngeal cancer, colorectal cancer, cervical cancer, prostate cancer, breast cancer, ovarian cancer, malignant melanoma, lung adenocarcinoma, liver cancer, or renal cancer, and the composition may contain cancer cells derived from one type or a plurality types of these cancers. The solid cancer is preferably colorectal cancer or breast cancer. The breast cancer may be triple-negative breast cancer, which is negative for any marker of estrogen, prolactin, and Her2.

The cancer cells may be cancer cells collected from the subject (i.e., cancer cells collected from the subject to be administered with the composition of the present embodiment) or cancer cells collected from a subject other than the subject to be administered with the composition of the present embodiment, but the former is preferred. Regardless of the subject from which the cancer cells are collected, preferably, the cancer cells are collected in the form of a cancer tissue, and then, for example, the tissue is degraded by enzymatic treatment into a single cell state.

The cancer cells may be subjected to a pretreatment, such as purification of the cancer cells, as necessary.

The subject is preferably a mammalian individual (living body). The mammal is not particularly limited, but examples include a human and a non-human mammal. Examples of the non-human mammal include mouse, rat, guinea pig, hamster, cattle, goat, sheep, pig, monkey, dog, and cat. The site of administration is not particularly limited, and examples include an intradermal site, a subcutaneous site, a muscular layer, and a lymph node. The site of administration may be one or a plurality of these. Other examples of the site of administration include organs or tissues, such as the pancreas, the brain, the pharynx, the large intestine, the uterine cervix, the prostate, the mammary gland, the ovary, the skin, the lung, the liver, and the kidney. The site of administration may be one or a plurality of these organs or tissues.

The content of the cancer cells in the composition is not particularly limited as long as the effects of the composition of the present embodiment are not impaired.

For example, the lower limit can be 1.0 x 10⁵ cells/mL or more, 5.0 x 10⁵ cells/mL or more, 1.0 x 10⁶ cells/mL or more, 5.0 x 10⁶ cells/mL or more, 1.0 x 10⁷ cells/mL or more, 5.0 x 10⁷ cells/mL or more, 1.0 x 10⁸ cells/mL or more, 5.0 x 10⁸ cells/mL or more, 1.0 x 10⁹ cells/mL or more, 5.0 x 10⁹ cells/mL or more, 1.0 x 10¹⁰ cells/mL or more, 5.0 x 10¹⁰ cells/mL or more, or 1.0 x 10¹¹ cells/mL or more, and the upper limit can be 1.0 x 10¹² cells/mL or less, 5.0 x 10¹¹ cells/mL or less, 1.0 x 10¹¹ cells/mL or less, 5.0 x 10¹⁰ cells/mL or less, 1.0 x 10¹⁰ cells/mL or less, 5.0 x 10⁹ cells/mL or less, 1.0 x 10⁹ cells/mL or less, 5.0 x 10⁸ cells/mL or less, 1.0 x 10⁸ cells/mL or less, 5.0 x 10⁷ cells/mL or less, 1.0 x 10⁷ cells/mL or less, 5.0 x 10⁶ cells/mL or less, or 1.0 x 10⁶ cells/mL or less. In addition, these lower limits and upper limits may be freely combined to form a certain range, and the range may be, for example, 1.0 x 10⁵ cells/mL or more and 1.0 x 10¹² cells/mL or less, 5.0 x 10⁵ cells/mL or more and 5.0 x 10¹¹ cells/mL or less, 1.0 x 10⁶ cells/mL or more and 1.0 x 10¹¹ cells/mL or less, 5.0 x 10⁶ cells/mL or more and 5.0 x 10¹⁰ cells/mL or less, 1.0 x 10⁷ cells/mL or more and 1.0 x 10¹⁰ cells/mL or less, 5.0 x 10⁷ cells/mL or more and 5.0 x 10⁹ cells/mL or less, or 1.0 x 10⁸ cells/mL or more and 1.0 x 10⁹ cells/mL or less. When expressed in terms of weight per mL of the composition, the content may be from 1 ng/mL to 1000 mg/mL, from 1 µg/mL to 1000 mg/mL, or from 1 mg/mL to 1000 mg/mL.

The dose of the composition of the present embodiment is not particularly limited as long as the effects of the composition are not impaired, and the dose can be appropriately adjusted depending on the age, sex, or body weight of the subject, the type or stage of cancer, the route of administration, and the like. For example, in the case of parenteral administration to a human adult (body weight 60 kg), the composition having a content described above may be administered daily at 1 ng to 1000 mg, at 1 µg to 1000 mg, or at 1 mg to 1000 mg. The composition may be administered in a single dose or in multiple doses.

The composition of the present embodiment is parenterally administered using a needleless injector. Examples of the parenteral administration include intradermal administration, subcutaneous administration, and intramuscular administration, and the route of administration may be one or a plurality of these routes.

The composition of the present embodiment may be in a liquid form, and examples of the dosage form include an injection.

The composition of the present embodiment may contain the cancer cells that express DAMPs alone, or may be used in combination with a carrier or an additive. Examples of the carrier include a pharmacologically acceptable solvent or diluent. For example, water, saline, or a buffer solution is used for preparation of a liquid composition. Examples of the additive include a stabilizer, a pH adjuster, a thickener, an antioxidant, an isotonic agent, a buffer, a solubilizing agent, a suspending agent, a preservative, a cryoprotectant, a freezing protectant, a lyophilization protectant, and a bacteriostatic agent. The composition of the present embodiment can be produced by a method known to those skilled in the art.

The method for causing cancer cells to express DAMPs is not particularly limited, but DAMPs can be expressed, for example, by the method for inactivating cancer cells described below in the section <Composition Containing Inactivated Cancer Cells>.

### <Needleless Injector>

Another embodiment of the present disclosure is a needleless injector for administering the composition to the subject, the injector including:
a storage section containing the composition;
a nozzle section communicating with the storage section, the nozzle section having an ejection port for ejecting the composition toward the subject; and
a pressurization section for pressurizing the composition contained in the storage section during an operation, thereby ejecting the composition from the ejection port toward the subject.

The needleless injector of the present embodiment is also an embodiment of the needleless injector in the section <Composition Containing Cancer Cells That Express Damage-Associated Molecular Patterns (DAMPs)>.

In the needleless injector of the present embodiment, the term "tip end side" means a side on which the ejection port through which the composition is ejected from the needleless injector is disposed, and the term "base end side" means a side opposite to the tip end side in the needleless injector, and these terms do not limitedly indicate a specific portion or position.

In the needleless injector of the present embodiment, a drive section applies ejection energy to eject the composition toward the subject. The "ejection" by the needleless injector of the present embodiment is achieved by pressurizing the composition stored in the storage section by the pressurization section using the ejection energy by the drive section, and thus allowing the composition to flow through a flow path of the storage section. The ejection energy may be ejection energy used in a needleless injector known in the related art, and for example, combustion energy of an explosive or the like, generation energy of a gas generating agent or the like, electric energy of a piezoelectric element or the like, or mechanical energy of a spring or the like can be used, or energy obtained by appropriately combining these forms of energy can be used.

In the case of using the combustion energy of an explosive as the ejection energy, examples of the explosive include any one of an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), and an explosive containing aluminum and iron oxide (AFO), or an explosive containing a plurality of these explosives in combination. Such an explosive is characterized in that the combustion product thereof is gas at high temperature but does not contain a gas component at ordinary temperature, and thus the combustion product is condensed immediately after the ignition. Thus, in a pressurization process for ejection of the composition, the temperature of the combustion product at the time of pressurization can be shifted by the combustion of an ignition agent to near ordinary temperature in a short period of time after the pressure applied to the composition reaches a first peak ejection force.

In the case of using the generation energy of a gas generating agent as the ejection energy, the gas generating agent can be a single base smokeless explosive (e.g., a single base smokeless explosive containing 98 mass% of nitrocellulose, 0.8 mass% of diphenylamine, and 1.2 mass% of potassium sulfate), or any of various gas generating agents used in gas generators for air bags and gas generators for seat belt pretensioners.

In the needleless injector of the present embodiment, the pressurization section pressurizes the composition stored in the storage section during the operation, which causes the composition to be ejected from the ejection port toward the subject.

The pressurization by the pressurization section is not particularly limited as long as the system is not destroyed, for example, the storage section is not destroyed, and pressurization conditions in a common needleless injector can be employed.

In this context, the pressure means a pressure in the storage section. The method for measuring the pressure is not particularly limited, and for example, the pressure can be measured as follows. That is, as in the measurement method described in JP 2005-21640 A, the pressure is measured by a method in which an ejection force is applied in a dispersed manner to a diaphragm of a load cell disposed downstream of a nozzle and an output from the load cell is collected by a data collection device via a detection amplifier and is stored as an ejection force (N) per unit time. The thus-measured ejection pressure is divided by the area of an ejection port 31a of the needleless injector to calculate the ejection pressure. The measurement value obtained by the internal pressure measurement of the storage section is equivalent to the ejection pressure, and the ejection pressure can be regarded as the pressure inside the storage section.

The ejection energy by the drive section is transmitted to a plunger through a piston, and the plunger slides in the storage section, whereby the composition stored in the storage section is pushed out along the flow path formed in the nozzle section, and finally ejected from the ejection port toward the subject.

The composition may or may not be stored in the storage section from the beginning. In a case where the composition is not stored in the storage section, the composition can be stored in the storage section by aspirating the composition into the storage section through a nozzle having an ejection port. Employing the configuration that requires the storing operation into the storage section allows administration of any required composition to the subject. Thus, in the needleless injector of the present embodiment, a syringe section and an injector body may be detachably configured.

In the present embodiment, the administration to a subject with a needleless injector may be administration to the subject by jet injection.

The term "jet injection" in the present disclosure refers to an injection without using an injection needle, the injection being characterized in that the composition is ejected from an ejection port toward a subject to form a through hole penetrating the boundary between the inside and the outside of the subject, and a high-pressure ultrafine liquid flow capable of administering the composition into the subject is generated through the through hole. For example, the jet injection may refer to injection characterized by generating a high-pressure ultrafine liquid flow capable of penetrating the skin of the subject. Alternatively, in the case of administering the composition directly to an organ during abdominal surgery of the subject, the jet injection may refer to injection characterized by generating a high-pressure ultrafine liquid flow capable of penetrating the organ.

The needleless injector of the present embodiment can be obtained by storing the composition in the storage section in a typical needleless injector. Examples of such a typical needleless injector include the needleless injector described in WO 2019/156238, the needleless injector described in WO 2019/156239, the needleless injector described in WO 2019/156237, and the needleless injector described in JP 5989039 B. These are needleless injectors capable of jet injection, but in addition to these, there are many commercially available needleless injectors capable of the jet injection. Examples thereof include Straits (Pharmajet), Tropis (Pharmajet), Vitajet (Bioject Medical Technologies Inc.), Biojector 2000 (Bioject Medical Technologies Inc.), Bioject Zetajet (Bioject Medical Technologies Inc.), Glide (Glide Pharma), MediJector Vision (Antares Inc.), Sumaval DosePro (Zogenix Inc.), SQ Pen (Bespak), and Injex (Equidyne).

With reference to the drawings, an injector 1 (needleless injector) will be described below as an example of the needleless injector of the present embodiment. The configuration of the following embodiment is an example, and the needleless injector is not limited to the configuration of the present embodiment. The terms "tip end side" and "base end side" are used as terms indicating a relative positional relationship in the longitudinal direction of the injector 1. The term "tip end side" indicates a position close to the tip end of the injector 1 to be described below, that is, a position close to an ejection port 31a, and the term "base end side" indicates a direction opposite to the "tip end side" in the longitudinal direction of the injector 1, that is, a direction toward a side of a drive section 7. The present example is an example of using combustion energy of an explosive ignited by an ignition device as ejection energy for pressurization, but the present embodiment is not limited to this example.

### (Configuration of Injector 1)

FIG. 1 is a diagram illustrating a schematic configuration of the injector 1 and is also a cross-sectional view of the injector 1, taken along its longitudinal direction. The injector 1 is formed by attaching an injector assembly 10 to a housing (injector housing) 2, and the injector assembly 10 is formed by integrally assembling a subassembly including a syringe section 3 and a plunger 4 and a subassembly including an injector body 6, a piston 5, and a drive section 7.

As described above, the injector assembly 10 is configured to be attachable and detachable to and from the housing 2. A storage section 32 formed between the syringe section 3 and the plunger 4 included in the injector assembly 10 is filled with the composition. The injector assembly 10 is a unit that is disposed after every ejection of the composition. Meanwhile, a battery 9 that supplies power to an igniter 71 included in the drive section 7 of the injector assembly 10 is included on the housing 2 side. The power is supplied from the battery 9 through wiring between an electrode on the housing 2 side and an electrode on the drive section 7 side of the injector assembly 10 when a user performs an operation of pressing a button 8 provided on the housing 2. The electrode on the housing 2 side and the electrode on the drive section 7 side of the injector assembly 10 have shapes and positions designed to come into contact with each other automatically when the injector assembly 10 is attached to the housing 2. The housing 2 is a unit that can be repeatedly used as long as power that can be supplied to the drive section 7 remains in the battery 9. In the housing 2, when the battery 9 runs out of power, only the battery 9 is exchanged, and the housing 2 may continue to be used.

The details of the injector assembly 10 will next be described. First of all, a description is given on the subassembly including the syringe section 3 and the plunger 4. In the syringe section 3, the storage section 32 is formed as a space in which the composition can be stored. More specifically, as illustrated in FIG. 1, the plunger 4 is disposed slidably along an inner wall surface extending in the axial direction of the syringe section 3, and the storage section 32 is defined by the inner wall surface of the syringe section 3 and the plunger 4. In addition, the syringe section 3 includes a nozzle section 31 communicating with the storage section 32, and the ejection port 31a is formed on the tip end side of the nozzle section 31. The nozzle section 31 is a flow path whose cross-sectional area gradually decreases from the storage section 32 side toward the ejection port 31a side, and the flow path is configured to guide the composition filled in the storage section 32 to the ejection port 31a. In the example illustrated in FIG. 1, the shape on the tip end side of the plunger 4 substantially matches the shape of the nozzle section 31.

Next, the subassembly including the injector body 6, the piston 5, and the drive section 7 will be described. The piston 5 is made of, for example, metal and is configured to be pressurized by a combustion product (combustion gas) produced by the igniter 71 of the drive section 7 and to slide in a through hole formed inside the injector body 6. The injector body 6 is a substantially cylindrical member, and the piston 5 is contained therein slidably along the inner wall surface extending in the axial direction of the injector body 6. The piston 5 may be made of a resin, and in such a case, a metal may be used in combination for a portion required to have heat resistance or pressure resistance. In addition, as illustrated in FIG. 1, the piston 5 is integrally connected with the plunger 4.

Next, the drive section 7 will be described. As illustrated in FIG. 1, the drive section 7 is fixed on the base end side with respect to the through hole in the injector body 6. The drive section 7 includes the igniter 71, which is an electric igniter. The igniter 71 is disposed to face the interior of the through hole in the injector body 6, and an ignition agent is contained in the igniter 71. As the ignition agent, various types of explosives can be employed as described above. The ignition agent can be contained, for example, in an explosive cup formed of an appropriate thin metal.

Next, how the injector 1 having the above configuration is operated will be described. As illustrated in FIG. 1, in a state where the injector assembly 10 is attached to the housing 2, the composition is aspirated from the ejection port 31a of the nozzle section 31. This allows the composition to be filled in the storage section 32. In this state, for example, with the ejection port 31a of the injector 1 being in contact with a subject, when a user performs an operation of pressing the button 8 provided on the housing 2, this serves as a trigger and actuation power is supplied from the battery 9 to the igniter 71 of the drive section 7, and thus the igniter 71 is actuated. When the igniter 71 is actuated, the ignition agent is ignited and thus combusted, and combustion products (e.g., flame and combustion gas) are generated. As a result, for example, an explosive cup of the igniter 71 is ruptured, and the combustion gas of the ignition agent is released into the through hole in the injector body 6. This rapidly increases the pressure in the through hole of the injector body 6, and the piston 5 is pressed toward the tip end side of the injector body 6. As a result, the piston 5 slides along the inner wall surface of the through hole in the injector body 6 toward the tip end side. As described above, the plunger 4 is integrally connected with the piston 5, and thus the plunger 4 also slides along the inner wall surface of the syringe section 3 in conjunction with the piston 5. That is, when the plunger 4 is pushed toward the nozzle section 31 located on the tip end side of the syringe section 3, the volume of the storage section 32 storing the composition is reduced, and the composition is rapidly pressurized accordingly. As a result, the composition filled in the storage section 32 is pushed into the nozzle section 31 and is ejected from the ejection port 31a at a high pressure. Thus, the composition can be administered to the subject.

Although an additional explosive component is not particularly placed in the injector body 6 illustrated in FIG. 1, a gas generating agent or the like, which is combusted by a combustion product generated by the explosive combustion at the igniter 71 and generates gas, can be placed in the igniter 71 or the through hole of the injector body 6 to adjust a change in pressure applied to the composition through the piston 5. The configuration in which the gas generating agent is placed in the igniter 71 is a known technique as disclosed in WO 01-031282, JP 2003-25950 A, and the like. One example of the gas generating agent is a single base smokeless explosive containing 98 mass% of nitrocellulose, 0.8 mass% of diphenylamine, and 1.2 mass% of potassium sulfate. Various types of gas generating agents used in a gas generator for an air bag and a gas generator for a seat belt pretensioner can be used. A combustion completion time of the gas generating agent can be changed by adjusting the dimensions, size, shape, and particularly, surface shape of the gas generating agent when the gas generating agent is placed in the through hole, which allows a change in pressure applied to the composition to be adjusted to a desired change, that is, a change that enables the composition to appropriately reach the subject. In the present embodiment, a gas generating agent to be used as necessary is also included in the drive section 7. In the present embodiment, the plunger 4 and the piston 5 constitute the "pressurization section".

### <Composition Containing Inactivated Cancer Cells>

Another embodiment of the present disclosure is a composition containing inactivated cancer cells, the composition being administered to a subject with a needleless injector.

In the present embodiment, inactivating cancer cells refers to inducing death of immunogenic cells containing DAMPs such as HMGB1 accumulated therein by any technique. The thus-inactivated cancer cells typically express DAMPs described above in the section <Composition Containing Cancer Cells Expressing Damage-Associated Molecular Patterns (DAMPs)>.

In the present embodiment, the degree of inactivation of the cancer cells may be 10% or more, 20% or more, 30% or more, 40% or more, 50% or more, 60% or more, 70% or more, 80% or more, 90% or more, 95% or more, 98% or more, or 99% or more of all the cancer cells contained in the composition, or the degree of inactivation may be 100%.

The degree of inactivation of the cancer cells can be determined by, for example, performing MTS assay, which is a method known to those skilled in the art, 24, 48, and 72 hours after the cell inactivation treatment, and observing the death or growth state of the cells. Specifically, for example, when viable cells can be observed 24 hours after the cell inactivation treatment and viable cells cannot be observed 72 hours after the cell inactivation treatment, it can be determined that complete inactivation is achieved, that is, 100% of all the cancer cells contained in the composition are inactivated. Meanwhile, when viable cells can be observed 72 hours after the cell inactivation treatment, it can be determined that the inactivation is incomplete. For example, when 50% of all the cancer cells contained in the composition can be observed 72 hours after the cell inactivation treatment, it can be determined that the degree of inactivation is 50%.

The technique for inactivating the cancer cells is not particularly limited as long as it can inactivate the cancer cells. Examples of the technique include radiation treatment, drug treatment, and heat treatment, and the technique may be one or a plurality of these treatments. A combined treatment of radiation treatment and heat treatment is preferably performed.

In the case of using radiation treatment as the technique for inactivating the cancer cells, examples of the radiation treatment include treatment with X-rays, treatment with heavy particle beams such as carbon ion beams, treatment with alpha rays, treatment with beta rays, or treatment with gamma rays. In the case of treatment with X-rays, the dose of X-rays is not particularly limited as long as the cancer cells can be inactivated. For example, the lower limit may be 12 Gy or more, 25 Gy or more, 50 Gy or more, or 100 Gy or more, and the upper limit may be 100 Gy or less, 50 Gy or less, or 25 Gy or less. These upper and lower limits may be freely combined, and the dose range may be, for example, 12 Gy or more and 100 Gy or less, 25 Gy or more and 100 Gy or less, 50 Gy or more and 100 Gy or less, 12 Gy or more and 50 Gy or less, 25 Gy or more and 50 Gy or less, or 12 Gy or more and 25 Gy or less.

The cell count is preferably adjusted before radiation treatment. The cell count may be adjusted to, for example, from 1 x 10⁵ cells/mL to 1 x 10⁷ cells/mL, or from 5 x 10⁵ cells/mL to 5 x 10⁶ cells/mL, or may be adjusted to 1 x 10⁶ cells/mL.

In the case of using drug treatment as the technique for inactivating the cancer cells, examples of the drug treatment include treatment with a drug that causes apoptosis or necroptosis, such as mitomycin c, doxycycline, or B/B homodimerizer. Specifically, in the case of using mitomycin c, for example, mitomycin c is added to a solution containing the cancer cells to achieve a final concentration of mitomycin c of 1.5 µg/mL to 150 µg/mL (e.g., 15 µg/mL), and the cancer cells can be treated in a water bath at 37°C for 2 hours. In the case of using doxycycline, the treatment can be performed by, for example, adding doxycycline to a solution containing the cancer cells to achieve a final concentration of doxycycline of 0.1 mg/mL to 10 mg/mL (e.g., 1 mg/mL) and culturing the cells for 24 hours. In the case of using B/B homodimerizer, the treatment can be performed by, for example, adding B/B homodimerizer to a solution containing the cancer cells to achieve a final concentration of B/B homodimerizer of 1 nM to 100 nM (e.g., 10 nM) and culturing the cells for 24 hours. The drug concentration, reaction temperature, reaction time, and the like when each of the drugs is used can be appropriately changed.

In the case of using heat treatment as the technique for inactivating the cancer cells, for example, a suspension in which the cells are suspended in PBS to a concentration of 1 x 10⁷ cells/mL or less is placed in a microcentrifuge tube (1.5 mL), and can be treated on a dry bath incubator at 42°C to 55°C (e.g., 45°C) for 30 minutes. The reaction temperature, reaction time, and the like can be appropriately changed.

In the present embodiment, the cancer cells may be cultured for a certain period after the inactivation. Culturing for a certain period can accumulate DAMPs in the inactivated cancer cells. Here, the culturing for a certain period is not particularly limited. For example, the lower limit of the culturing period may be 12 hours or more, 24 hours or more, 48 hours or more, or 72 hours or more, and the upper limit of the curing period may be 24 hours or less, 48 hours or less, or 72 hours or less. These upper and lower limits may be freely combined, and the culturing period may be, for example, 12 hours or more and 24 hours or less, 12 hours or more and 48 hours or less, 12 hours or more and 72 hours or less, 24 hours or more and 48 hours or less, 24 hours or more and 72 hours or less, or 48 hours or more and 72 hours or less.

Culture conditions for the inactivated cancer cells are not particularly limited as long as the cancer cells can be cultured, and conditions, such as a medium to be used, temperature, and humidity, are not limited.

In the present embodiment, the cancer tissue collected from the subject is preferably degraded into a single cell state and then inactivated by radiation treatment or the like and cultured. However, the cancer tissue may be inactivated by radiation treatment or the like and cultured in a state where the cancer tissue is only fragmented or sliced without being degraded into a single cell state or in a state of the cancer tissue as it is.

For the description of DAMPs, the needleless injector, the effect by the administration, the cancer cells, the subject, the content of the cancer cells in the composition, the dose of the composition, the route of administration, the form, the dosage form, and the like, reference can be made to the description in the above section

### <Composition Containing Cancer Cells That Express Damage-Associated Molecular Patterns (DAMPs)>.

### <Composition Containing Cancer Cells Inactivated by Radiation Exposure and Heat Treatment>

Another embodiment of the present disclosure is a composition containing cancer cells inactivated by radiation exposure and heat treatment, the composition being administered to a subject with a needle-equipped injector.

When a combined treatment of radiation treatment and heat treatment is performed as the technique for inactivating the cancer cells, DAMPs in the cancer cells are efficiently released to the outside of the cancer cells not only in the case of administering to a subject with a needleless injector, but also in the case of administering to the subject with a needle-equipped injector. Then, DAMPs released from the cancer cells cause local inflammation, leading to an immune response. Presumably, this results in enhanced phagocytosis of dendritic cells and efficient activation of cellular immunity by the administered composition. Thereafter, the composition provides not only a local effect at the administration site of the composition, but also activation of cellular immunity to cancer cells present at a distance from the administration site.

That is, administering the composition containing cancer cells subjected to a combined treatment of radiation treatment and heat treatment to a subject with a needle-equipped injector can provide an effect similar to the abscopal effect with good reproducibility and can enhance the effect similar to the abscopal effect.

Thus, administering the composition of the present embodiment to a subject with a needle-equipped injector is useful in cancer treatment, cancer prevention, and the like. An effect similar to the abscopal effect can be expected even in inoperable cases or colorectal cancer cases for which radiotherapy is impossible, and thus administering the composition of the present embodiment to a subject with a needle-equipped injector is useful.

In the case of using radiation treatment as the technique for inactivating the cancer cells, examples of the radiation treatment include treatment with X-rays, treatment with heavy particle beams such as carbon ion beams, treatment with alpha rays, treatment with beta rays, or treatment with gamma rays. In the case of treatment with X-rays, the dose of X-rays is not particularly limited as long as the cancer cells can be inactivated. For example, the lower limit may be 12 Gy or more, 25 Gy or more, 50 Gy or more, or 100 Gy or more, and the upper limit may be 100 Gy or less, 50 Gy or less, or 25 Gy or less. These upper and lower limits may be freely combined, and the dose range may be, for example, 12 Gy or more and 100 Gy or less, 25 Gy or more and 100 Gy or less, 50 Gy or more and 100 Gy or less, 12 Gy or more and 50 Gy or less, 25 Gy or more and 50 Gy or less, or 12 Gy or more and 25 Gy or less.

The cell count is preferably adjusted before radiation treatment. The cell count may be adjusted to, for example, from 1 x 10⁵ cells/mL to 1 x 10⁷ cells/mL, or from 5 x 10⁵ cells/mL to 5 x 10⁶ cells/mL, or may be adjusted to 1 x 10⁶ cells/mL.

In the case of using heat treatment as the technique for inactivating the cancer cells, for example, a suspension in which the cells are suspended in PBS to a concentration of 1 x 10⁷ cells/mL or less is placed in a microcentrifuge tube (1.5 mL), and can be treated on a dry bath incubator at 42°C to 55°C (e.g., 45°C) for 30 minutes. The reaction temperature, reaction time, and the like can be appropriately changed.

The composition of the present embodiment is parenterally administered using a needle-equipped injector. Examples of the parenteral administration include intradermal administration, subcutaneous administration, and intramuscular administration, and the route of administration may be one or a plurality of these routes. The type of the needle-equipped injector is not particularly limited, and the gauge and length of the needle and the shape of the needle tip to be used may be selected from those commonly used depending on the administration site.

For the description of DAMPs, the cancer cells, the subject, the content of the cancer cells in the composition, the dose of the composition, the form, the dosage form, and the like, reference can be made to the description in the above section <Composition Containing Cancer Cells That Express Damage-Associated Molecular Patterns (DAMPs)>. For the definition of the inactivation of the cancer cells, the degree of inactivation, and culture after the inactivation, reference can be made to the description in the above section

### <Composition Containing Inactivated Cancer Cells>.

### <Composition Containing Cancer Cells for Treating or Preventing Cancer>

Any of the compositions described in the above section <Composition Containing Cancer Cells That Express Damage-Associated Molecular Patterns (DAMPs)>, <Composition Containing Inactivated Cancer Cells>, or <Composition Containing Cancer Cells Inactivated by Radiation Exposure and Heat Treatment> can be used for treating or preventing a cancer. That is, any of these compositions can be used as a pharmaceutical composition.

In the present embodiment, the expression "cancer has been treated" can be determined by, for example, a decrease in the number of cancer cells, a decrease in the volume of cancer, or complete disappearance of cancer in a subject administered with the composition, as compared with a control subject not administered with the composition or the subject before administration of the composition.

In the present embodiment, the prevention of cancer may be, for example, prevention of cancer in a subject inexperienced with cancer, or may be prevention of recurrence or metastasis after solid cancer removal.

The composition of the present embodiment can also be used as a vaccine for treating or preventing a cancer.

The cancer to be treated or prevented is preferably a cancer from which the cancer cells contained in the composition of the present embodiment are derived. Examples of the cancer include pancreatic cancer, brain tumor, pharyngeal cancer, colorectal cancer, cervical cancer, prostate cancer, breast cancer, ovarian cancer, malignant melanoma, lung adenocarcinoma, liver cancer, or renal cancer, and the cancer may be one or a plurality of these. Specifically, when the cancer to be treated or prevented is breast cancer, the cancer cells contained in the composition of the present embodiment are preferably cancer cells derived from breast cancer to be treated or prevented.

The cancer cells may be cancer cells collected from the subject (i.e., cancer cells collected from the subject to be administered with the composition of the present embodiment) or cancer cells collected from a subject other than the subject to be administered with the composition of the present embodiment, but the former is preferred.

For the description of DAMPs, the needleless injector, the effect by the administration, the cancer cells, the subject, the content of the cancer cells in the composition, the dose of the composition, the route of administration, the form, the dosage form, the inactivation, and the like, reference can be made to the description in the above section <Composition Containing Cancer Cells That Express Damage-Associated Molecular Patterns (DAMPs)>, <Composition Containing Inactivated Cancer Cells>, or <Composition Containing Cancer Cells Inactivated by Radiation Exposure and Heat Treatment>.

Other examples of the present embodiment include, for example, the following:
- a method for treating or preventing a cancer, the method including administering a composition containing cancer cells that express damage-associated molecular patterns (DAMPs) to a subject with a needleless injector;
- a method for treating or preventing a cancer, the method including administering a composition containing inactivated cancer cells to a subject with a needleless injector;
- a composition for use in treating or preventing a cancer, the composition containing cancer cells that express damage-associated molecular patterns (DAMPs), and being administered to a subject with a needleless injector;
- a composition for use in treating or preventing a cancer, the composition containing inactivated cancer cells, and being administered to a subject with a needleless injector;
- use of a composition containing cancer cells that express damage-associated molecular patterns (DAMPs) in manufacturing a medicine for treating or preventing a cancer, the medicine being administered to a subject with a needleless injector;
- use of a composition containing inactivated cancer cells in manufacturing a medicine for treating or preventing a cancer, the medicine being administered to a subject with a needleless injector;
- a method for treating or preventing a cancer, the method including administering a composition containing cancer cells inactivated by radiation exposure and heat treatment to a subject with a needle-equipped injector;
- a composition for use in treating or preventing a cancer, the composition containing cancer cells inactivated by radiation exposure and heat treatment, and being administered to a subject with a needle-equipped injector; and
- use of a composition containing cancer cells inactivated by radiation exposure and heat treatment in manufacturing a medicine for treating or preventing a cancer, the medicine being administered to a subject with a needle-equipped injector.

### <Composition Containing Cancer Cells for Activating Cellular Immunity to Cancer>

Any of the compositions described in the above section <Composition Containing Cancer Cells That Express Damage-Associated Molecular Patterns (DAMPs)>, <Composition Containing Inactivated Cancer Cells>, or <Composition Containing Cancer Cells Inactivated by Radiation Exposure and Heat Treatment> can be used for activating cellular immunity to cancer. That is, any of these compositions can be used as a pharmaceutical composition.

Administering the composition of the present embodiment to a subject with a needleless injector can efficiently activate cellular immunity to cancer as compared with the case of using a needle-equipped injector.

That is, unlike the case of using a needle-equipped injector, the use of a needleless injector causes the cancer cells contained in the composition to be damaged by shear force at the same time as the administration, and thus DAMPs in the cancer cells are efficiently released to the outside of the cancer cells. Then, DAMPs released from the cancer cells cause local inflammation, leading to an immune response. Presumably, this results in enhanced phagocytosis of dendritic cells and efficient activation of cellular immunity by the administered composition. Thereafter, the composition provides not only a local effect at the administration site of the composition, but also activation of cellular immunity to cancer cells present at a distance from the administration site.

For the description of DAMPs, the needleless injector, the effect by the administration, the cancer cells, the subject, the content of the cancer cells in the composition, the dose of the composition, the route of administration, the form, the dosage form, the inactivation, and the like, reference can be made to the description in the above section <Composition Containing Cancer Cells That Express Damage-Associated Molecular Patterns (DAMPs)>, <Composition Containing Inactivated Cancer Cells>, or <Composition Containing Cancer Cells Inactivated by Radiation Exposure and Heat Treatment>.

Other examples of the present embodiment include, for example, the following:
- a method for activating cellular immunity to cancer, the method including administering a composition containing cancer cells that express damage-associated molecular patterns (DAMPs) to a subject with a needleless injector;
- a method for activating cellular immunity to cancer, the method including administering a composition containing inactivated cancer cells to a subject with a needleless injector;
- a composition for use in activating cellular immunity to cancer, the composition containing cancer cells that express damage-associated molecular patterns (DAMPs), and being administered to a subject with a needleless injector;
- a composition for use in activating cellular immunity to cancer, the composition containing inactivated cancer cells, and being administered to a subject with a needleless injector;
- use of a composition containing cancer cells that express damage-associated molecular patterns (DAMPs) in manufacturing a medicine for activating cellular immunity to cancer, the medicine being administered to a subject with a needleless injector;
- use of a composition containing inactivated cancer cells in manufacturing a medicine for activating cellular immunity to cancer, the medicine being administered to a subject with a needleless injector;
- a method for activating cellular immunity to cancer, the method including administering a composition containing cancer cells inactivated by radiation exposure and heat treatment to a subject with a needle-equipped injector;
- a composition for use in activating cellular immunity to cancer, the composition containing cancer cells inactivated by radiation exposure and heat treatment, and being administered to a subject with a needle-equipped injector; and
- use of a composition containing cancer cells inactivated by radiation exposure and heat treatment in manufacturing a medicine for activating cellular immunity to cancer, the medicine being administered to a subject with a needle-equipped injector.

### <Kit for Treating or Preventing Cancer>

Another embodiment of the present disclosure is a kit for treating or preventing a cancer, the kit including the above-described needleless injector and the above-described cancer cell-containing composition. Alternatively, another embodiment of the present disclosure is a kit for treating or preventing a cancer, the kit including the above-described needle-equipped injector and the above-described cancer cell-containing composition.

Such a kit can optionally include a buffer solution, a diluent, or the like in addition to the needleless injector or the needle-equipped injector and the cancer cell-containing composition.

### Examples

The present disclosure will next be described in more detail by way of Examples, but it goes without saying that the scope of the present disclosure is not limited only to the Examples.

### <Example 1: Administration of Radiation-Treated Breast Cancer Cells to Mice>

4T1 cells (obtained from American Type Culture Collection (ATCC)), i.e., breast cancer cells were treated with radiation at a dose of 100 Gray with Gammacell (registered trademark) 40 Exactor and then cultured in RPMI 1640 complete medium for 24 hours. After the elapse of 24 hours, the cells were harvested and cryopreserved in Stem Cellbanker until use. In this case, the 4T1 cells were treated with radiation, but the cancer cells were insufficiently inactivated.

Before administration of the above cancer cells to mice (Balb/c, 6 weeks old, male or female), the cells stored in Stem Cellbanker were thawed and washed twice with PBS. 1 x 10⁶ cancer cells (suspended in 30 µL of PBS) were subcutaneously administered to the buttock of each mouse with a needle-equipped injector or a needleless injector. One week after the administration, it was determined whether a tumor developed at the administration site of the cancer cells.

As a result, when the needle-equipped injector (indicated as Normal syringe in figures) was used, a tumor developed in all mice (FIG. 2, N = 4). In contrast, when the needleless injector (indicated as DD in figures) was used, no tumor development was observed in all mice (FIG. 2, N = 4).

Also, before administration of the above cancer cells to mice (Balb/c, 6 weeks old, male or female, n = 4), the cells stored in Stem Cellbanker were thawed and washed twice with PBS. 5 x 10⁵ cancer cells (suspended in 30 µL of PBS) were subcutaneously administered to the buttock of each mouse with a needle-equipped injector or a needleless injector. Four days after the administration, the second administration of the cancer cells was performed in the same manner. Ten days after completion of the second inoculation, a cancer-specific immune response was analyzed by the following method.

Firstly, the spleen removed from each of the mice was ground to harvest splenocytes. The splenocytes were treated with AKC lysing buffer (Gibco) for 5 minutes and washed twice with PBS at 5 x 10⁵ cells/mL. In addition, 4T1 cells were treated in the presence of Mitomycin C (Nacalai, 15 µg/mL) in a water bath at 37°C for 3 hours and then washed twice with PBS.

The splenocytes treated with AKC lysing buffer and the 4T1 cells treated with Mitomycin C were mixed at a cell count ratio of 10: 1 and cultured in RPMI 1640 complete medium with 50 µM 2-mercaptoethanol (Nacalai) for 48 hours.

After the culture, the splenocytes were harvested, 5 x 10⁵ splenocytes were seeded in an IFN-gamma ELIspot plate together with 2 x 10³ 4T1 cells, and cultured for 48 hours. After the culture, the ELIspot plate was subjected to a color reaction by a method known to those skilled in the art.

As a result, in the group of administration of the radiation-treated breast cancer cells with a needleless injector (indicated as DD in the figure), a larger number of color reactions were observed than in the group of administration of the radiation-treated breast cancer cells with a needle-equipped injector (indicated as Normal syringe in the figure) (FIG. 3).

That is, unlike the case of using a needle-equipped injector, the use of a needleless injector caused the breast cancer cells to be damaged by shear force at the same time as the administration, and thus DAMPs in the breast cancer cells were efficiently released to the outside of the breast cancer cells. Then, DAMPs released from the breast cancer cells caused local inflammation, which led to an immune response.

This resulted in enhanced phagocytosis of dendritic cells and efficient activation of cellular immunity by the administered composition. Thereafter, the composition provided not only a local effect at the administration site of the composition, but also activation of cellular immunity to cancer cells present at a distance from the administration site.

### <Example 2: Administration of Radiation-Treated Colorectal Cancer Cells to Mice>

MC38 cells (obtained from American Type Culture Collection (ATCC)), i.e., colorectal cancer cells were treated with radiation at a dose of 25 Gray with Gammacell (registered trademark) 40 Exactor and then cultured in DMEM complete medium (DMEM + 10% FBS + penicillin/streptomycin) for 24 hours. After the elapse of 24 hours, the cells were harvested and cryopreserved in Stem Cellbanker until use.

Before administration of the above cancer cells to mice (C57/B6, 6 weeks old, male or female), the cells stored in Stem Cellbanker were thawed and washed twice with PBS. 1 x 10⁶ cancer cells (suspended in 30 µL of PBS) were subcutaneously administered to the buttock of each mouse with a needle-equipped injector or a needleless injector. For control, colorectal cancer cells subjected to no radiation exposure were administered to the buttocks of mice with a needle-equipped injector or a needleless injector. One week after the administration, it was determined whether a tumor developed at the administration site of the cancer cells.

As a result, in the group of administration of the non-radiation-treated cells with a needle-equipped injector (indicated as NL in the figure), a tumor developed in all mice (FIG. 4, N = 5). In contrast, in the group of administration of the radiation-treated cells with a needle-equipped injector (indicated as ND in the figure), in the group of administration of the non-radiation-treated cells with a needleless injector (indicated as DDL in the figure), and in the group of administration of the radiation-treated cells with a needleless injector (indicated as DDD in the figure), no tumor development was observed in all mice (FIG. 4, N = 5 in each group).

Also, before administration of the above cancer cells to mice (C57/B6, 6 weeks old, male or female, n = 4), the cells stored in Stem Cellbanker were thawed and washed twice with PBS. 1 x 10⁶ cancer cells (suspended in 30 µL of PBS) were subcutaneously administered to the buttock of each mouse with a needle-equipped injector or a needleless injector. Four days after the administration, the second administration of the cancer cells was performed in the same manner. Ten days after completion of the second inoculation, a cancer-specific immune response was analyzed by the following method.

Firstly, the spleen removed from each of the mice was ground to harvest splenocytes. The splenocytes were treated with AKC lysing buffer (Gibco) for 5 minutes and washed twice with PBS at 5 x 10⁵ cells/mL. In addition, MC38 cells were treated in the presence of Mitomycin C (Nacalai, 15 µg/mL) in a water bath at 37°C for 3 hours and then washed twice with PBS.

The splenocytes treated with AKC lysing buffer and the MC38 cells treated with Mitomycin C were mixed at a cell count ratio of 10: 1 and cultured in RPMI 1640 complete medium with 50 µM 2-mercaptoethanol (Nacalai) for 48 hours.

After the culture, the splenocytes were harvested, 5 x 10⁵ splenocytes were seeded in an IFN-gamma ELIspot plate together with 2 x 10³ MC38 cells, and cultured for 48 hours. After the culture, the ELIspot plate was subjected to a color reaction by a method known to those skilled in the art.

As a result, in the group of administration of the radiation-treated colorectal cancer cells with a needleless injector (indicated as DDD in the figure), a larger number of color reactions were observed than in the group of administration of the non-radiation-treated cells with a needle-equipped injector (indicated as NL in the figure), in the group of administration of the radiation-treated cells with a needle-equipped injector (indicated as ND in the figure), and in the group of administration of the non-radiation-treated cells with a needleless injector (indicated as DDL in the figure) (FIG. 5). That is, unlike the case of using a needle-equipped injector, the use of a needleless injector caused the colorectal cancer cells to be damaged by shear force at the same time as the administration, and thus DAMPs in the colorectal cancer cells were efficiently released to the outside of the colorectal cancer cells. Then, DAMPs released from the colorectal cancer cells caused local inflammation, which led to an immune response.

This resulted in enhanced phagocytosis of dendritic cells and efficient activation of cellular immunity by the administered composition. Thereafter, the composition provided not only a local effect at the administration site of the composition, but also activation of cellular immunity to cancer cells present at a distance from the administration site.

### <Example 3: Examination of HMGB1 Expression Levels in Various Cancer Cells after Radiation Exposure>

4T1 cells (obtained from American Type Culture Collection (ATCC)), i.e., breast cancer cells were treated with radiation at a dose of 100 Gray with Gammacell (registered trademark) 40 Exactor and then cultured in RPMI 1640 complete medium for 24 hours. In addition, MC38 cells (obtained from American Type Culture Collection (ATCC)), i.e., colorectal cancer cells were treated with radiation at a dose of 25 Gray with Gammacell (registered trademark) 40 Exactor and then cultured in DMEM complete medium (DMEM + 10% FBS + penicillin/streptomycin) for 24 hours.

These cultured cells were treated with RIPA buffer (obtained from Nacalai) to extract proteins. 2.5 µg of the extracted proteins were separated by 5 to 15% polyacrylamide electrophoresis (PAGE). The electrophoresed proteins were then transferred to a membrane and reacted with detection antibodies by Western blotting. The primary antibodies used were HMGB-1 (biolegend, direct-blot, clone 3E8, cat; 651411) and β-actin (cell signaling, clone 8H10D10, cat3700S), and the secondary antibody used was anti-mouse IgG (cytiva, cat: NA931V). After the antibody reaction, chemiluminescence was detected using immunoStar (wako); i.e., a chemiluminescent reagent. The results are illustrated in FIG. 6. When 4T1 cells were used, the expression level of HMGB-1 in the 25 Gy-irradiated cancer cells was 1.04 times that in Mock, and the expression level of HMGB-1 in the 50 Gy-irradiated cancer cells was 1.22 times that in Mock. When MC38 cells were used, the expression level of HMGB-1 in the 12 Gy-irradiated cancer cells was 1.41 times that in Mock, and the expression level of HMGB-1 in the 25 Gy-irradiated cancer cells was 1.7 times that in Mock.

### <Example 4: Administration of Radiation and Heat Combined Treated Colorectal Cancer Cells to Mice with Needle-Equipped Injector>

### • Acquisition of Radiation-Treated Cells

MC38 cells (obtained from American Type Culture Collection (ATCC)), i.e., colorectal cancer cells were cultured in DMEM complete medium (DMEM + 10% FBS + 1% penicillin/streptomycin). The cells were cultured to 80% confluence and treated with radiation at a dose of 25 Gy with Gammacell (registered trademark) 40 Exactor. Then, 2 mL of a trypsin EDTA solution was added, and the culture was incubated at 37°C for 5 minutes to detach the cells from the plate. The cells were harvested by centrifugation and discarding the supernatant. The harvested cells were cultured for 24 hours. After the elapse of 24 hours, the cells were harvested and added to Cellbanker, i.e., a cryopreservation solution to achieve a cell concentration of 5 x 10⁶ cells/mL and stored at -80°C.

### • Acquisition of Heat-Treated Cells

MC38 cells (obtained from American Type Culture Collection (ATCC)), i.e., colorectal cancer cells were cultured in DMEM complete medium (DMEM + 10% FBS + 1% penicillin/streptomycin). The cells were cultured to 80% confluence and washed with 10 mL of PBS. 2 mL of a trypsin EDTA solution was added, the culture was incubated at 37°C for 5 minutes, and then it was confirmed that the cells were detached from the plate. Then, the cells were harvested by centrifugation and discarding the supernatant. DMEM complete medium (DMEM + 10% FBS + penicillin/streptomycin) was added and stirred to prepare a cell suspension. Then, the cell suspension was heat-treated at 45°C for 30 minutes. Then, the heat-treated cells were cultured for 24 hours. After the elapse of 24 hours, the cells were harvested and added to Cellbanker to achieve a cell concentration of 5 x 10⁶ cells/mL and stored at -80°C.

### • Acquisition of Cells Subjected to Combined Treatment with Radiation and Heat

MC38 cells (obtained from American Type Culture Collection (ATCC)), i.e., colorectal cancer cells were cultured in DMEM complete medium (DMEM + 10% FBS + 1% penicillin/streptomycin). The cells were cultured to 80% confluence and treated with radiation at a dose of 25 Gray with Gammacell (registered trademark) 40 Exactor. Then, the medium was discarded, and the cells were washed with 10 mL of PBS. 2 mL of a trypsin EDTA solution was added, the culture was incubated at 37°C for 5 minutes, and then it was confirmed that the cells were detached from the plate. Then, the cells were harvested by centrifugation and discarding the supernatant. DMEM complete medium (DMEM + 10% FBS + 1% penicillin/streptomycin) was added and stirred to prepare a cell suspension. Then, the cell suspension was heat-treated at 45°C for 30 minutes. Then, the cells were harvested by centrifugation. The harvested cells were cultured in DMEM complete medium (DMEM + 10% FBS + 1% penicillin/streptomycin) for 24 hours. After the elapse of 24 hours, the cells were harvested and added to Cellbanker to achieve a cell concentration of 5 x 10⁶ cells/mL and stored at -80°C.

Also, before administration of the cancer cells treated by the above method to mice (C57/B6, 6 weeks old, male or female, n = 4), the cells were thawed and then washed twice with PBS.

1 x 10⁶ cancer cells (suspended in 100 µL of PBS) treated by the above three methods were subcutaneously administered to the buttocks of different mice, respectively, with a needle-equipped injector. Four days after the administration, the second administration of the cancer cells was performed in the same manner. Ten days after completion of the second inoculation, a cancer-specific immune response was analyzed by the following method.

Firstly, the spleen removed from each of the mice was ground to harvest splenocytes. The splenocytes were treated with AKC lysing buffer (Gibco) for 5 minutes and washed twice with PBS at 5 x 10⁵ cells/mL. In addition, MC38 cells were treated in the presence of Mitomycin C (Nacalai, 15 µg/mL) in a water bath at 37°C for 3 hours and then washed twice with PBS.

The splenocytes treated with AKC lysing buffer and the MC38 cells treated with Mitomycin C were mixed at a cell count ratio of 10: 1 and cultured in RPMI 1640 complete medium with 50 µM 2-mercaptoethanol (Nacalai) for 48 hours.

After the culture, the splenocytes were harvested, 5 x 10⁵ splenocytes were seeded in an IFN-gamma ELIspot plate together with 2 x 10³ MC38 cells, and cultured for 48 hours. After the culture, the ELIspot plate was subjected to a color reaction by a method known to those skilled in the art.

As a result, spots of IFN γ-producing cells were observed in each of the mouse groups of inoculation of the radiation-treated colorectal cancer cells and the heat-treated colorectal cancer cells (indicated as Radiation and Heat, respectively, in FIG. 7). Furthermore, a larger number of color reactions were observed in the mouse group of inoculation of the colorectal cancer cells subjected to the combined treatment; i.e., the radiation treatment and the subsequent heat treatment (indicated as Mix in FIG. 7) (FIG. 7). That is, a combined effect by the radiation treatment and the heat treatment was confirmed.

### <Example 5: Administration of Radiation and Heat Combined Treated Colorectal Cancer Cells to Mice with Needle-Equipped and Needleless Injectors>

Cells subjected to combined treatment with radiation and heat were acquired by the following technique. MC38 cells (obtained from American Type Culture Collection (ATCC)), i.e., colorectal cancer cells were cultured in DMEM complete medium (DMEM + 10% FBS + 1% penicillin/streptomycin). The cells were cultured to 80% confluence and treated with radiation at a dose of 25 Gray with Gammacell (registered trademark) 40 Exactor. Then, the medium was discarded, and the cells were washed with 10 mL of PBS. 2 mL of a trypsin EDTA solution was added, the culture was incubated at 37°C for 5 minutes, and then it was confirmed that the cells were detached from the plate. Then, the cells were harvested by centrifugation and discarding the supernatant. DMEM complete medium (DMEM + 10% FBS + 1% penicillin/streptomycin) was added and stirred to prepare a cell suspension. Then, the cell suspension was heat-treated at 45°C for 30 minutes. Then, the cells were harvested by centrifugation. The harvested cells were cultured in DMEM complete medium (DMEM + 10% FBS + 1% penicillin/streptomycin) for 24 hours. After the elapse of 24 hours, the cells were harvested and added to Cellbanker to achieve a cell concentration of 5 x 10⁶ cells/mL and stored at -80°C.

Also, before administration of the cancer cells treated by the above method to mice (C57/B6, 6 weeks old, male or female, n = 4), the cells were thawed and then washed twice with PBS.

Using a needle-equipped injector or a needleless injector, 1 x 10⁶ cancer cells (suspended in 100 µL of PBS for a needle-equipped injector; or suspended in 30 µL of PBS for a needleless injector) subjected to the combined treatment by the above method were subcutaneously administered to the buttocks of different mice, respectively. Four days after the administration, the second administration of the cancer cells was performed in the same manner. Ten days after completion of the second inoculation, a cancer-specific immune response was analyzed by the following method.

Firstly, the spleen removed from each of the mice was ground to harvest splenocytes. The splenocytes were treated with AKC lysing buffer (Gibco) for 5 minutes and washed twice with PBS at 5 x 10⁵ cells/mL. In addition, MC38 cells were treated in the presence of Mitomycin C (Nacalai, 15 µg/mL) in a water bath at 37°C for 3 hours and then washed twice with PBS.

The splenocytes treated with AKC lysing buffer and the MC38 cells treated with Mitomycin C were mixed at a cell count ratio of 10:1 and cultured in RPMI 1640 complete medium with 50 µM 2-mercaptoethanol (Nacalai) for 48 hours.

After the culture, the splenocytes were harvested, 5 x 10⁵ splenocytes were seeded in an IFN-gamma ELIspot plate together with 2 x 10³ MC38 cells, and cultured for 48 hours. After the culture, the ELIspot plate was subjected to a color reaction by a method known to those skilled in the art.

As a result, a larger number of color reactions were observed in the mouse group of inoculation of the colorectal cancer cells subjected to the combined treatment by the above method with a needleless injector (indicated as DD in FIG. 8) than in the mouse group of inoculation of the colorectal cancer cells with a needle-equipped injector (indicated as Normal syringe in FIG. 8) (FIG. 8). Thus, the results indicated that, also in the case of using the cells subjected to the combined treatment with radiation and heat, administration with a needleless injector induces cellular immunity more efficiently.

### Reference Signs List

1 Injector, 2 Housing, 3 Syringe section, 4 Plunger, 5 Piston, 6 Injector body, 7 Drive section, 8 Button, 9 Battery, 10 Injector assembly, 31 Nozzle section, 31a Ejection port, 32 Storage section, 71 Igniter

## Claims

1. A composition comprising cancer cells that express damage-associated molecular patterns (DAMPs), the composition being administered to a subject with a needleless injector.

2. The composition according to claim 1, wherein the DAMPs contain at least HMGB1.

3. A composition comprising inactivated cancer cells, the composition being administered to a subject with a needleless injector.

4. The composition according to claim 3, wherein the cancer cells are inactivated by radiation exposure, drug administration, or heat treatment.

5. The composition according to claim 4, wherein the cancer cells are inactivated by radiation exposure and heat treatment.

6. The composition according to claim 3, wherein the cancer cells are cultured for a certain period after the inactivation.

7. The composition according to any one of claims 1 to 6, wherein the cancer cells contained in the composition are cancer cells derived from a solid cancer.

8. The composition according to claim 7, wherein the solid cancer is pancreatic cancer, brain tumor, pharyngeal cancer, colorectal cancer, cervical cancer, prostate cancer, breast cancer, ovarian cancer, malignant melanoma, lung adenocarcinoma, liver cancer, or renal cancer.

9. The composition according to any one of claims 1 to 6, wherein the cancer cells are cancer cells collected from the subject.

10. The composition according to any one of claims 1 to 6, wherein the composition is for treating or preventing a cancer.

11. The composition according to claim 10, wherein the cancer to be treated or prevented is a cancer from which the cancer cells contained in the composition are derived.

12. The composition according to any one of claims 1 to 6, wherein the composition is for activating cellular immunity to cancer.

13. A kit for treating or preventing a cancer, the kit comprising a needleless injector and the composition described in any one of claim 1 to 6.

14. A composition comprising cancer cells inactivated by radiation exposure and heat treatment, the composition being administered to a subject with a needle-equipped injector.

15. A kit for treating or preventing a cancer, the kit comprising a needle-equipped injector and the composition described in claim 14.
